Europäisches Patentamt

European Patent Office

Office européen des brevets

(11) Publication number: **0 137 504**
**B1**

## (12) EUROPEAN PATENT SPECIFICATION

(45) Date of publication of patent specification: **16.01.91**

(21) Application number: **84112218.7**

(22) Date of filing: **11.10.84**

(51) Int. Cl.⁵: **C 12 N 15/04,** C 12 N 13/00, H 01 S 1/00

(54) Method and apparatus of implanting living cells with a foreign substance.

(30) Priority: **13.10.83 JP 191378/83**
**13.10.83 JP 191379/83**

(43) Date of publication of application:
**17.04.85 Bulletin 85/16**

(45) Publication of the grant of the patent:
**16.01.91 Bulletin 91/03**

(84) Designated Contracting States:
**CH DE FR GB LI**

(56) References cited:
**FR-A-2 224 902**

**SCIENCE, vol. 213, 31st July 1981, pages 505-512, AAAS, Lancaster, US; M.W. BERNS et al.: "Laser microsurgery in cell and developmental biology"**

**PATENTS ABSTRACTS OF JAPAN, vol. 7, no. 167 (C-177)1312r, 22nd July 1983; & JP-A-58 76 091 (MITSURU FURUSAWA) 09-05-1983**

(73) Proprietor: **RIKAGAKU KENKYUSHO**
**2-1 Hirosawa**
**Wako-shi Saitama-ken (JP)**
(73) Proprietor: **Science & Technology Agency**
**No. 2-1, Kasumigaseki 2-chome**
**Tokyo (JP)**

(72) Inventor: **Kasuya, Takahiro c/o RIKAGAKU KENKYUSHO**
**No. 2-1, Hirosawa**
**Wako-shi Saitama-ken (JP)**
Inventor: **Ikawa, Yoji c/o RIKAGAKU KENKYUSHO**
**No. 2-1, Hirosawa**
**Wako-shi Saitama-ken (JP)**
Inventor: **Tsukakoshi, Motoo c/o RIKAGAKU KENKYUSHO**
**No. 2-1, Hirosawa**
**Wako-shi Saitama-ken (JP)**
Inventor: **Kurata, Shunichi**
**No. 3-3-1, Kata-cho**
**Fuchu-shi Tokyo (JP)**
Inventor: **Nomiya, Yoshio c/o RIKAGAKU KENKYUSHO**
**No. 2-1, Hirosawa**
**Wako-shi Saitama-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP  0 137 504  B1**

(74) Representative: **Lorenz, Eduard et al**
**Rechtsanwälte Eduard Lorenz - Bernhard Seidler**
**Margrit Seidler - Dipl.-Ing. Hans-K. Gossel Dr. Ina**
**Philipps - Dr. Paul B. Schäuble Dr. Siegfried**
**Jackermeier**
**Widenmayerstrasse 23 D-8000 München 22 (DE)**

## Description

This invention relates to a method of incorporating a foreign substance into living cells.

In the field of analysis of gene expression the behaviour of those genes which determine the character of a living thing is studied by incorporating selected genes into living cells and by examining the transformation of the implanted cells.

For example, a DNA can be extracted from cancerous cells, and the substance is divided into minute fragments of different sizes. These fragments are classified in terms of size. Then, DNA fragments of different sizes are incorporated into living cells. Cancerous cells being detected among those implanted cells, DNA fragments which are supposed to be the cause for cancer can be determined in term of size.

A prior art implantation method comprises the steps of: putting living cells in a solution containing DNA fragments; making a small hole in each living cell with the aid of a fine needle under the optical microscope; allowing DNA fragments to get in the host cell through its hole; and confining the fragments in the host cell when the hole in the cell heals (see Japanese Patent Application No. 56-171 347, published under the number of 58-76 091). Another prior art implantation method comprises the steps of: precipitating a DNA in the form of calcium phosphate in a culture medium; and making use of the phagocytosis of living cells to incorporate DNA precipitation into the host cells.

These prior art implantation methods, however, have major defects. The former implantation method requires a skilled manipulation of the needle. Otherwise, no holes can be made without injuring living cells. Also the work is tedious and laborious although the implantation succeeds at a relatively high rate. Still disadvantageously certain cells do not permit hole-making with any needles.

The latter implantation method is capable of handling a great number of cells at one time. The rate at which DNA fragments are incorporated into host cells, however, remains very low, say one in ten thousand (1/10,000) at best. The rate at which the character of the implanted cells is transformed is very small, and accordingly a very large number of cells need to be implanted with DNA fractions. This demand cannot be met. Also, disadvantageously the method requires addition of calcium phosphate of so high a concentration that the additive tends to injure living cells. A later analysis must be conducted with much care.

The inventors have found that a living cell when exposed to a laser of appropriate energy will be partly and temporarily modified to be permeable to a foreign substance, thereby allowing the substance to enter the living cell and confining the same in the living cell when recovering to the original condition. This invention is based on the discovery.

One object of this invention is to provide a method of implanting a foreign substance such as DNA in a very large number of cells with the maximum efficiency possible.

Another object of this invention is to provide an apparatus for implanting a foreign substance such as DNA in a very large numbers of cells in the minimum possible time.

Still another object of this invention is to provide an apparatus for making temporarily living cells permeable to a foreign substance without necessarily subjecting living cells to a special treatment.

To attain these objects an in vitro implantation method according to this invention comprises the steps of: exposing each living cell to a fine laser beam to bring at least one part of the cell surface to the condition in which the host cell allows a foreign substance such as DNA, protein or any other biopolymer to enter the host cell; and putting partly and temporarily modified living cells in a condition such that they meet with fractions of the foreign substance suspended in a medium.

Advantageously a laser beam has good directionality, and can be focused to a spot of minimal diameter using an optical microscope. These properties make it possible to make a minute "hole" (submicron in diameter) in a host cell. A pulse laser beam can be used then, the temperature rise of the cell caused by exposure to the laser can be advantageously suppressed by reducing the pulse duration of the laser, thereby preventing the cell from being thermally killed. Lasers produce monochromatic irradiation. The wavelength of the irradiation can be selected as appropriate for the purpose of making "holes" in a given type host cell, considering the optical characteristics of the cell wall and the biomembrane as well as those of a foreign substance to be incorporated.

The strength of a laser also can be adjusted over a wide range using appropriate electric control. The focusing depth in the host cell can be easily controlled with appropriate optical means. These together provide a great advantage to the microsurgery to which this invention is to be applied. An apparatus for making "holes" in living cells according to this invention uses a laser microbeam appropriate for the purpose of conducting a microsurgery on a living cell without injuring its self-healing capability.

The length of time for which cells are exposed to a laser microbeam should be controlled so as to be long enough to make "holes" in the cells, but not enough to thermally kill the cells. The exposure time can be controlled by using a series of laser pulses each having a duration controlled appropriately for the purpose or by scanning with a laser microbeam in an appropriate length of time a field in which a large number of living cells are suspended in a solution. Also, a laser microbeam can be thrown to a plurality of selected portions of a single cell among those floating still in a solution.

Thus, an apparatus which is known in general from the reference "Science", Vol. 213, p. 505-513, can be used for making "holes" in living cells and comprises: a laser source for providing a pulse on continuous wave laser microbeam, an optical system for projecting a laser microbeam to living cells, and means for monitoring living suspended cells in a solution. Another microsurgery apparatus comprises: a laser source, an optical system for projecting a laser microbeam to living cells, means for monitoring living cells suspended in a solution, means for determining the position of a cell selected among those appearing in the field of said monitoring means, and means responsive to a start signal from said positions determining means for controlling the supply of the laser from said laser system.

Still another microsurgery apparatus comprises: a laser source, an optical system for projecting a laser microbeam to living cells, means for monitoring living cells suspended in a solution, means for determining the position of a cell selected among those appearing in the field of said monitoring means, means responsive to cell position signals from said position determining means for storing, and means for controlling the laser microbeam and directing the same to selected cells one after another.

Other objects and advantages of this invention will be understood from the following description of preferred embodiments of this invention which is made with reference to accompanying drawings:

Fig. 1 is a copy of microscopic photograph showing NRK cells planted with gene (Ecogpt) according to this invention;

Fig. 2 shows a sequence of microscopic photographs showing the formation of a hole in an NRK cell using a laser microbeam and the subsequent healing of the hole;

Fig. 3 is a copy of microscopic photograph showing punched cells (human red blood cell);

Fig. 4 is a schematic block diagram of a laser punching apparatus according to a first embodiment;

Fig. 5 is a perspective view of a laser microbeam deflector used in the laser punching apparatus of Fig. 4;

Fig. 6 is a schematic block diagram of a laser punching apparatus according to a second embodiment;

Fig. 7 is a schematic block diagram of a laser punching apparatus according to a third embodiment;

Fig. 8 is a schematic block diagram of a laser punching apparatus according to a fourth embodiment;

Fig. 9 is a schematic block diagram of a laser punching apparatus according to a fifth embodiment and

Fig. 10 is a copy of microscopic photograph showing details of portions of cells swept by a laser microbeam.

As described earlier an implantation method according to this invention comprises the steps of exposing each living host cell to a laser microbeam to make the surface of the host cells temporarily permeable to a foreign substance, i.e. to make "holes" in the host cells, and allowing these permeable cells to meet with fragments of a foreign substance, such that the foreign substance enters at least some of the permeable cells before these cells heal their holes, and confining the foreign substance in the host cells when their holes close.

There are a variety of modes in which fragments of a foreign substance can meet with permeable host cells and enter the cell through the holes. For example, cells and fragments of the foreign substance are suspended in a solution, and cells are exposed to a laser microbeam one after another, thus causing permeable cells to coexist with the fragments in the solution. In a second approach, a solution containing host cells is supplied in drops to another solution containing fragments of a foreign substance, and then each drop on the way to the lower solution is exposed and punched by a laser microbeam. Third, a liquid carrying host cells and fragments of a foreign substance is made to flow across the field of a pulse or continuous wave laser. This final mode is most appropriate for the purpose of handling a lot of host cells.

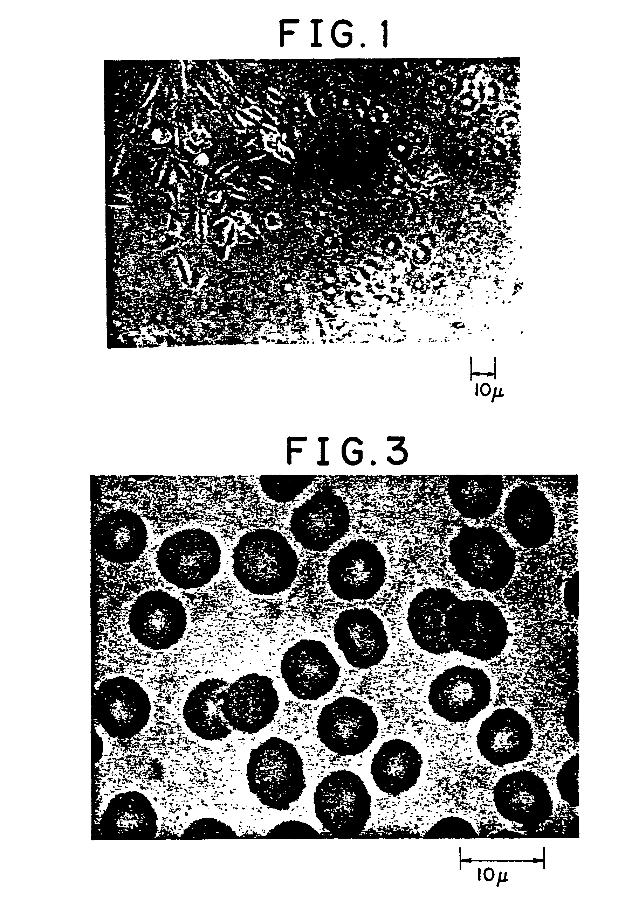

Referring to Fig. 1, living cells implanted with fragments of a foreign substance and dead cells which could not survive owing to no foreign substance implanted therein are shown under microscope. Specifically, cultivated NRK cells originating from the kidney of an Osborn Mendel rat were modified so as to be unable to survive without Ecogpt (Xanthine-guanine Phosporibosyl Transferase) being incorporated therein, and the NRK cells thus modified were put in an Ecogpt-containing medium (DMEM added with 10-percent unborn calf's blood serum). An infrared beam (wavelength $\lambda$ = 1.06 microns) from a laser device (YAG laser) was converted to an ultraviolet beam ($\lambda$ = 335 nanometers), and the ultraviolet beam was introduced into a laser microscope. Then, living cells floating in the medium in the field of the microscope were exposed to pulse laser beam having a pulse duration of 10 nanoseconds. The laser beam was thrown at the rate of ten pulses per second, and a lot of living cells were treated in the same condition. The result is shown in the left half of Fig. 1 whereas cells exposed to no laser shown in the right half of the drawing.

The cells exposed to the laser took fragments of the foreign substance and therefore, survived, but the cells that were not exposed to the laser could not take up fragments and therefore, died.

Living cells can heal their holes immediately after being made.

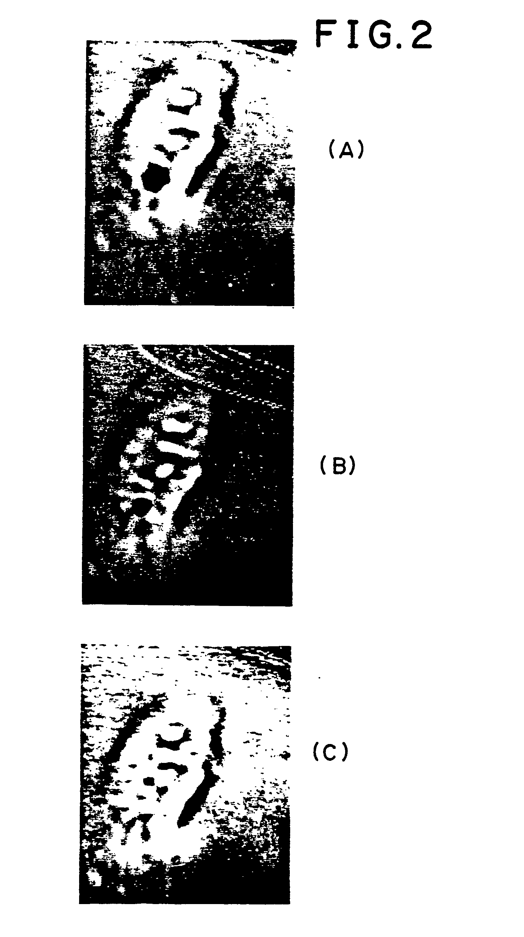

Fig. 2 is a photograph of a video sight showing NRKs immediately after being punched by a laser microbeam. Specifically, Fig. 2 (A) shows the appearance of cells at the instant they were punched; Fig. 2 (B) shows the appearance of cells immediately after being punched; and Fig. 2 (C) shows the appearance of cells after healing their hole (arrows indicating holes).

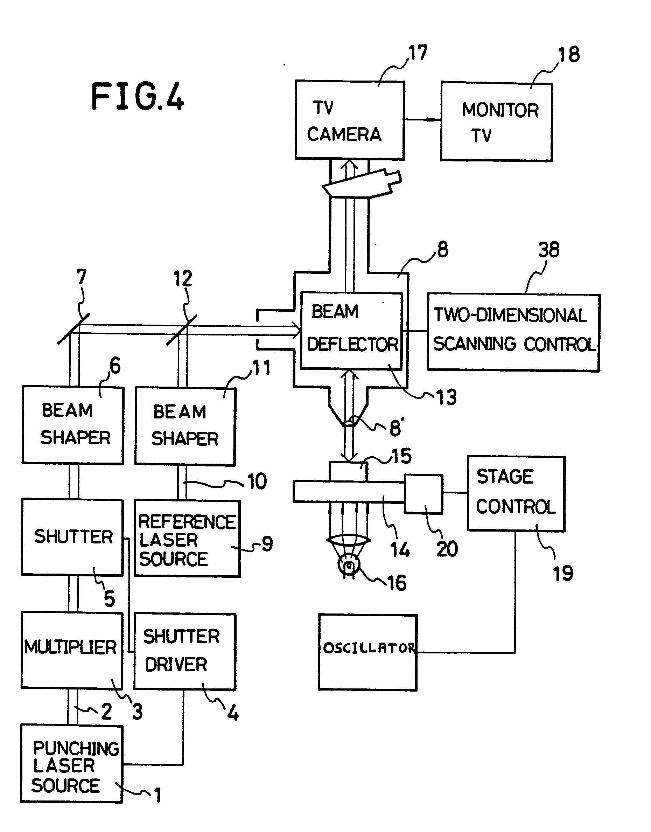

Fig. 3 shows human blood cells dyed and punched with a laser microbeam. These photographs show the appearance of living cells immediately after being punched, proving that a single cell can be punched at selected locations thanks to the good controllability of laser beams. As is apparent from the above the method of this invention permits punching for the sake of implantation of a foreign substance in the cell. Also, this invention can be equally applied to microsurgery of cells, as for instance breaking a particular minute organ in a single cell.

Application of this invention to the incorporation of genes into cells permits: production of useful substances in cells (for instance, synthesis of insulin or any other useful human substance in living cells; and improvement of domestic animals and agricultural products by substituting different genes for each other in different kinds of plants; or by incorporating good genes without recourse to fertilization).

Now, a cell-punching is described with reference to Fig. 4.

A laser beam ($\lambda = 1060$ nm) for punching living cells is thrown from a punching laser source 1 to a frequency multiplier 3 essentially composed of KDP or any other crystal appropriate for the purpose of converting to an ultraviolet ($\lambda = 335$ nm or $\lambda = 265$ nm). The ultraviolet laser beam passes through a shutter which is controlled by an associated shutter driver 4. Then, the laser beam 2 is shaped by a beam shaper 6. The laser beam shaped is changed in direction by reflector 7, and is directed to a microscope-and-beam combiner 8. On the other hand, a reference laser beam 10 functioning as a pilot or tracing beam (for instance, He-Ne laser, $\lambda = 633$ nm in the range of visible ray) is generated by a visible laser source 9. The reference laser 10 is shaped by a beam shaper 11, and then the shaped laser beam is reflected by a reflector 12 to travel toward the beam combiner 8 along with the punching laser beam 2. The punching and reference laser beams 2 and 10 after passing through a beam deflector 13 are combined by a condenser lens 8'. The combined laser beam is thrown to cells floating in a solution in which fractions of a foreign substance such as DNA are suspended. While cells are exposed to the laser beam, they are punched. A sample holder 15 is illuminated by a lamp 16 under the holder, thereby projecting an image of distribution of living cells in the sample holder to a TV camera 17 through the condenser lens 8', and producing a visible image of cell distribution on a TV monitor 18. A stage 14 carrying the sample holder 15 is composed of an X-Y stage which is driven by a stepping motor 20. The shutter 5 is closed to prevent the throwing of the punching laser beam to the sample holder 15, and then the visible laser beam 10 from the laser source 9 functions as a pilot beam, thus indicating on which place the punching laser beam is to be thrown. Otherwise, when combined with the punching laser beam 2 the visible laser beam 10 functions as a tracing beam, thus making visible the trace on which the punching laser beam travels.

In punching cells, the stage 14 is driven until the image of a congregation of cells appears in the field of the monitor 18. Then the shutter 5 is kept open, thereby permitting the continuous throwing of the punching laser beam 2 onto the sample holder 15. Then, cells are exposed to the punching laser beam 2 one after another simply by moving the stage 14. Fractions of a foreign substance floating in the vicinity of punched cells get in the "holes" of these punched cells. The living cells heal their holes in a few seconds, thus confining the foreign substance in the cells. As a result the living cells carry a particular gene. Moving the sample holder with respect to the still pulse or continuous move laser beam to cause the same to sweep the cell-floating area in the solution is most effective to treat a lot of cells within a relatively short time.

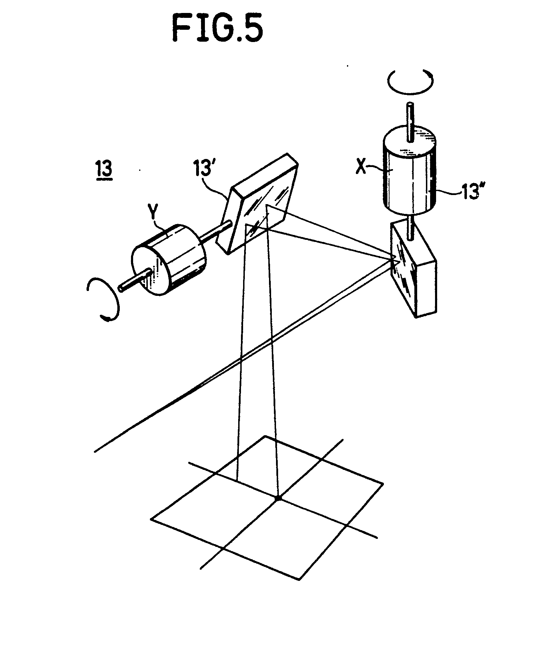

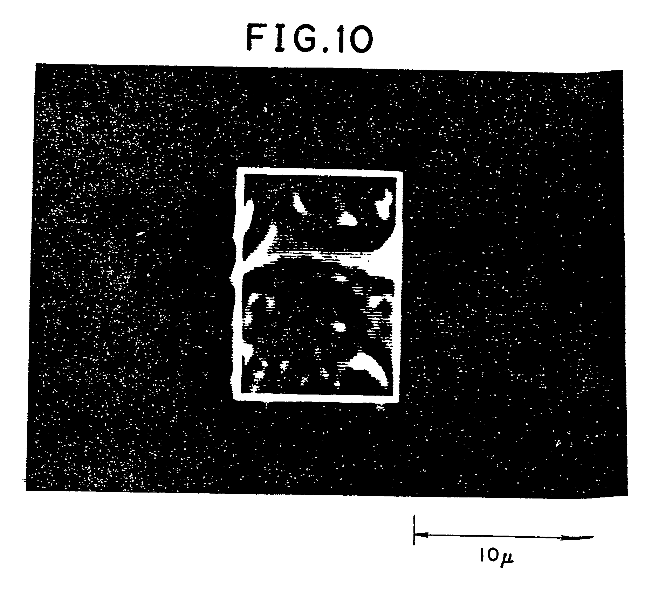

Fig. 5 shows the laser deflector 13 as comprising a combination of two galvanometers 13' and 13'' each equipped with a reflector. The laser deflector 13 is driven by an associated two-dimensional scanning control 38 so as to cause the visible laser beam 10 to scan a selected small area in the field of the sample holder. Then, resultant reflected rays, luminescence rays and scattered rays fall on the TV camera or a still camera after passing through the condenser 8' thereby producing a clear image showing, in detail, the inner structures of selected cells. Fig. 10 is a copy of microscopic photograph taken by sweeping with the visible laser beam, showing human red blood cells.

The area encircled with white line is the one swept by the visible laser beam 10, showing details of the inner structures of selected human red blood cells, in contrast with the rest area of the photograph illuminated by the lamp 16. Although the reason for providing such a clear detailed image of the inner structure of the cell is not known, it appears to the inventors that the laser after passing through the condenser lens focuses on a point at a determined depth, thereby causing the appearance of a clear image of the inner structure of the cell taken along the focal plane at the depth. Thus the cell punching apparatus equipped with a laser sweeping which permits the monitoring of modification of the inner structure of a cell punched and implanted with a foreign substance.

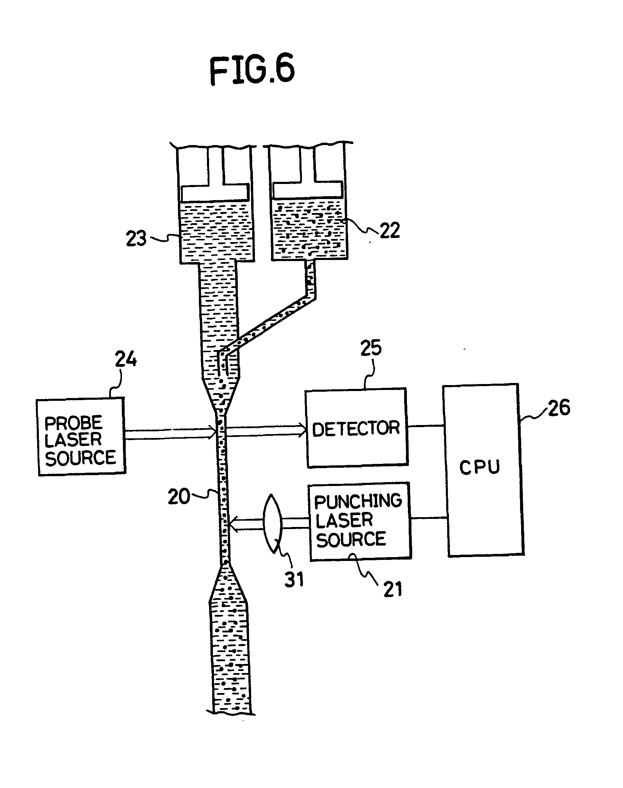

Fig. 6 shows a second cell punching apparatus useful in the method of this invention. As shown, cells descend one after another in a fine transparent tube 20 so that they are exposed to the punching laser beam from a laser source 21. Specifically, a solution 22 containing living cells and a protection liquid such as physiological saline 23 are fed to the fine tube 20. A probe laser beam is emitted by a probe laser source 24 to a detector 25, and passes through the descending flow upstream of the place at which cells are exposed to the punching laser beam. The detector 25 detects a cell passing by the detector to generate and sends a detection signal to a central processing unit 26, and then the central processing unit 26 times the start of the punching laser source 21, thus causing the punching laser beam to hit the descending cell to make a hole therein. Fragments of a foreign substance to be incorporated in cells such as DNA may be put in the solution 22 or the physiological saline 23. Thus, the cell punching apparatus can punch about 1000 cells per second. If use is made of a detector capable of determining the angle of diffusion over which the laser beam spreads when falling on a cell, cells can be classified in terms of size, and hence kind. Thus, it is possible to select and punch a particular kind of cells among different ones in a solution 22. The casting of the laser beam on cells may be controlled by controlling a shutter provided between the fine tube 20 and the laser source 21 rather than by controlling the punching laser source 21.

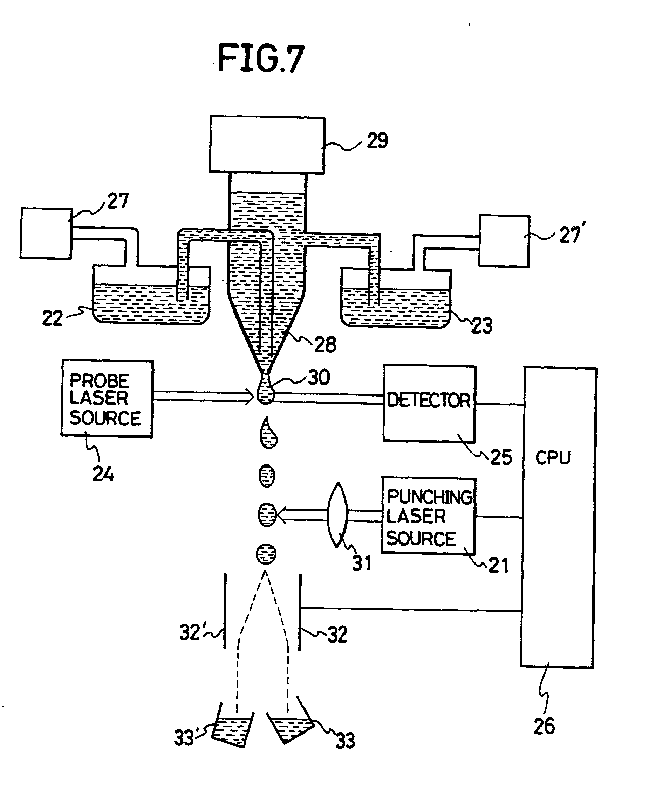

Fig. 7 shows a cell-punching apparatus useful in the invention. This apparatus is so designed that a solution containing living cells is supplied in drops across the punching laser beam. Specifically, a suspension 22 containing living cells, and a protection liquid 23 such as physiological saline are fed to a nozzle 28 under pressure by air pumps 27 and 27'. A mixture of suspension and protection liquid falls in drops 30 under the action of a supersonic nozzle vibrator 29, which may be composed of, for instance, a piezoelectric element. In operation, the fall of a drop 30 is detected by a probe laser falling on a detector 25, and then the detector 25 sends a detection signal to a central processing unit 26. Thus, the central processing unit 26 allows a punching laser source 21 to throw a punching laser beam at the instant the drop is about to cross the punching laser source 21, thereby making holes in cells in the drop.

When drops 30 are exposed to the probe laser, it is possible to determine which kind (or size) of cells are contained in each drop with the aid of a conventional laser analyzing system, and if drops are charged with electricity of which the polarity and/or quantity varies with the kind of the cell, and if these drops fall across the electric field between opposite electrodes 32 and 32', they will be classified in terms of the polarity and/or quantity of the electric charge, and will be put in different receptacles 33 and 33', thus classifying punched cells in terms of kind.

As an alternative, if the drops are charged with electricity of the same sign and quantity, the strength of the electric field may be varied with the kind of the punched cells. Fragments of a foreign substance to be incorporated into cells may be put in the suspension 22, the protection liquid 23 or in the receptacles 33 and 33'. The casting of the punching laser beam from the punching laser source 21 may be controlled by controlling a shutter (not shown) provided between the path of drops and the punching laser source 21 rather than by controlling the laser source 21. In some instances the continuous casting of the punching laser beam may be preferred.

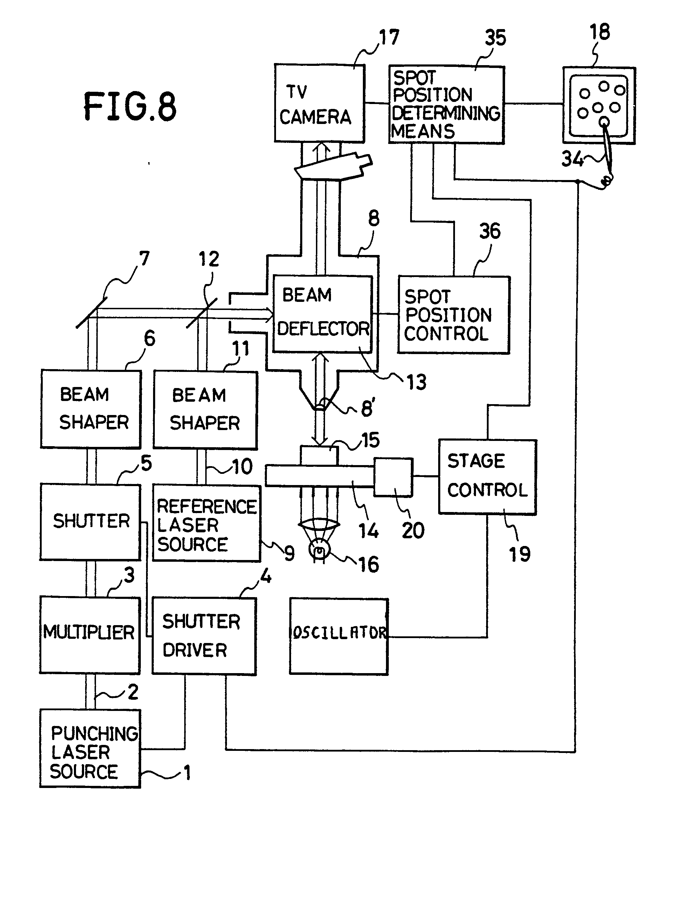

Fig. 8 shows a cell punching apparatus according to a fourth embodiment.

The cell punching apparatus of Fig. 8 is different from that of Fig. 4 in that the former is equipped with a light pen 34 for indicating cells appearing in the field of the TV monitor 18, an associated spot position determing means 35 for determining the coordinates of point of the monitor field on which the light pen is put and a spot position control 36 for controlling the laser deflector 13 so as to direct the laser beam to the same point as the light pen indicates. The spot position control 36 is responsive to a coordinate signal from the spot position determining means 35 for driving the laser deflector 13 to direct the laser beam to the position indicated by the light pen. In operation, the distribution of living cells in the sample holder 15 is watched by the monitor TV 18, and the light pen 34 is put on a selected part of a desired living cell selected among those appearing in the field of the TV monitor 18. The coordinate of the point indicated by the light pen 34 is determined by the spot position determining means 35. A position signal representing the position indicated by the light pen is directed from the spot position determining means 35 to the spot position control 36. Then, the spot position control 36 drives the laser deflector 13 to direct the laser beam 2 and 10 to the point indicated by the light pen 34.

At the same time as the light pen indicates a given position, the position determining means generates a start signal, and the shutter driver 4 is responsive to the start signal for opening the shutter 5 for a predetermined period. Thus, a corresponding number of laser pulses 2 are thrown onto the point indicated by the light pen 34. The punched cell allows fractions of a foreign substance to get therein, and then the cell heals its hole to confine the fraction therein as described earlier.

In this particular embodiment the spot position control 36 is used to drive the laser deflector 13 for throwing the laser beam to a given position. As an alternative the stage position control 19 is used to drive the stage 14 to attain the same effect.

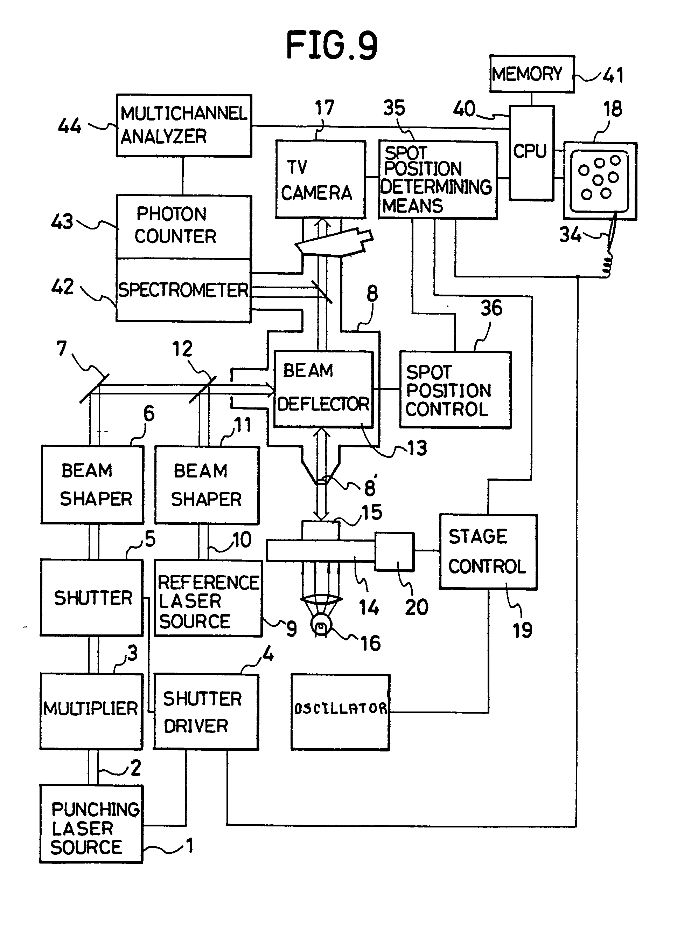

Fig. 9 shows a fifth cell punching apparatus useful in the invention.

A light pen 34 is used to indicate selected point or points on each of selected cells or every cell appearing in the field of a monitor TV 18, and a spot position determining means 35 determines the coordinates of the points indicated by the light pen 34. Then, signals representing these coordinates are directed to memory 41 through a central processing unit 40 so that the coordinates of the points indicated by the light pen are stored in the memory 41. These coordinates are read out one after another to input in the spot position control 36 under the control of the central processing unit 40. The spot position control 36 controls the laser deflector 13 in the same way as the cell punching apparatus of the fourth embodiment. On the other hand, the central processing unit 40 directs a drive signal to the shutter driver 4, thereby opening the shutter 5 for a predetermined period to throw a punching laser beam 2 to the points indicated by the light pen one after another. Thanks to the use of memory living cells appearing in the field of the monitor TV are punched in rapid succession. It is possible to make a decision as to whether a cell is present or not in terms of the amplitude of video-signal from the TV camera 17, and if the spot position detector 35 is designed to make such a decision, the cell punching will be completely automated. Specifically, the so designed spot position detector 35 may analyze video-signals from the TV camera 17, thereby determining the positions of cells appearing in the field of the TV camera, and then the position signals representing the positions of cells are directed to the memory 41 for storing. In this case a pattern identification means may be used to identify cells in terms of the contour of cell.

As shown in Fig. 9, a spectrometer 42, a photon-counter 43 and a multichannel analyzer 44 together constitute an analyzing system, which may be used as a monitor. Specifically, the optical system can make a decision as to whether a cell is present or not at a given coordinate (and in some instances a decision as to whether a cell core is present or absent at a given coordinate), in terms of spectrographic characteristics.

The embodiments described above use two different laser sources, that is, punching laser source and reference laser source. It, however, should be noted that if a continuous or non-pulse visible laser beam is used as a punching one, no reference laser beam is necessary because the spot on which the punching beam focuses is visible in the field of a TV monitor. Also, it should be noted that a shutter for controlling the throwing of the punching laser beam is not limited to the mechanical one, and that a conventional photo-switch may be used for the purpose. The expression, "fragments or fractions of a foreign substance" used herein is intended to include virus, every kind of protein and full genome of DNA.

Finally, in the examples and embodiments described herein above, selected portion or portions of each living cell are modified when exposed to a laser beam. This should not be understood as limitative. Indeed, the whole area of the living cell may be modified if use is made of a laser beam larger in diameter than the living cell.

## Claims

1. Method of implanting living cells in vitro with a foreign substance comprising the steps of:

a) exposing living cells to a laser beam to modify each living cell to be temporarily permeable to the foreign substance;

b) putting the cells and fragments with the foreign substance in a condition such that they may meet with each other.

2. Method according to claim 1 wherein said laser beam is a continuous wave or pulse laser.

3. Method according to claim 2 wherein the step "a" includes carrying said cells across said laser beam.

4. Method according to claim 2 wherein the step "a" includes sweeping a predetermined area with said continuous wave laser beam, and the step "b" includes putting a medium containing said living cells and fractions of said foreign substance in said predetermined area.

5. Method according to claim 2 wherein said medium is a liquid appropriate for the cultivation of said living cells.

6. Apparatus for carrying out the method of one of the claims 1-5 comprising:

a laser source;

an optical system for throwing a laser beam to each selected living cell;

a monitor for presenting the image of said selected cell;

means for determining the position at which each selected living cell stays in the field of said monitor;

means responsive to a signal from said cell-position determining means for throwing said laser beam from said laser source and

means responsive to a signal from said cell-position; determining means for directing said laser beam to a living cell staying at the position represented by said signal.

7. Apparatus for carrying out the method of one of the claims 1-5 comprising:

a laser source;

an optical system for throwing a laser beam to each selected living cell;

a monitor for presenting the image of said selected cell;

means for determining the position at which each selected living cell stays in the field of said monitor;

memory means responsive to signals from said cell-position determining means for throwing the positions of selected living cells and

means for reading pieces of information from said memory means for directing said laser beam to said selected cells one after another.

## Patentansprüche

1. In vitro durchgeführtes Verfahren zum Implantieren eines Fremdstoffes in lebende Zellen mit folgenden Schritten:

a) durch Bestrahlen von lebenden Zellen mit einem Laserstrahl wird jede Zelle derart modifiziert, daß sie zeitweilig für den Fremdstoff durchlässig ist;

b) die Zellen und die Fragmente mit dem Fremdstoff werden in einen Zustand versetzt, in dem sie miteinander in Berührung gelangen können.

2. Verfahren nach Anspruch 1, dadurch gekennzeichnet, daß der Laserstrahl ein kontinuierlicher oder getasteter Laserstrahl ist.

3. Verfahren nach Anspruch 2, dadurch gekennzeichnet, daß im Schritt "a" die Zellen quer durch den Laserstrahl bewegt werden.

4. Verfahren nach Anspruch 2, dadurch gekennzeichnev daß im Schritt "a" ein vorherbestimmter Bereich mit einem kontinuierlichen Laserstrahl bestrichen wird und daß im Schritt "b" ein die lebenden Zellen und Fragmente des Fremdstoffes enthaltendes Medium in den vorherbestimmten Bereich eingebracht wird.

5. Verfahren nach Anspruch 2, dadurch gekennzeichnet, daß das genannte Medium eine zum Züchten der lebenden Zellen geeignete Flüssigkeit ist.

6. Vorrichtung zum Durchführen des Verfahrens nach einem der Ansprüche 1 bis 5 mit

einer Laserquelle;

einem optischen System zum Richten eines Laserstrahls auf jede ausgewählte lebende Zelle;

einem Monitor zum Erzeugen eines Bildes der genannten ausgewählten Zelle;

einer Einrichtung zum Erfassen der Position der Stelle, an der jede ausgewählte lebende Zelle auf dem Bildschirm des Monitors erscheint;

einer Einrichtung, die auf Grund eines von der Zellenpositons-Erfassungeinrichtung abgegebenen Signals die Abgabe des Laserstrahls durch die Laserquelle bewirkt; und

einer Einrichtung, die auf Grund eines von der Zellenpositions-Erfassungseinrichtung kommenden Signals den Laserstrahl auf eine lebende Zelle richtet, die sich in der durch das genannte Signal dargestellten Position befindet.

7. Vorrichtung zum Durchführen des Verfahrens nach einem der Ansprüche 1 bis 5 mit

einer Laserquelle;

einem optischen System zum Richten eines Laserstrahls auf jede ausgewählte lebende Zelle;

einem Monitor zum Erzeugen eines Bildes der genannten ausgewählten Zelle;

einer Einrichtung zum Erfassen der Position der Stelle, an der jede ausgewählte lebende Zelle auf dem Bildschirm des Monitors erscheint;

einer Speichereinrichtung zum Speichern der positionen von ausgewählten lebenden Zellen auf Grund von von der Zellenpositons-Erfassungseinrichtung kommenden Signalen; und

einer Einrichtung zum Auslesen von Informationen aus der Speichereinrichtung und zum Richten des Laserstrahls auf aufeinanderfolgende einzelne ausgewählte Zellen.

## Revendications

1. Procédé pour implanter dans des cellules vivantes in vitro une substance étrangère comprenant les étapes.

a) d'exposition des cellules vivantes à un faisceau laser pour modifier chaque cellule vivante de manière qu'elle soit temporairement perméable à la substance étrangère;

b) de mise des cellules et fragments avec la substance étrangère dans un état tel qu'ils puissent se rencontrer.

2. Procédé selon la revendication 1 dans lequel ledit faisceau laser est un laser à ondes continues ou à impulsions.

3. Procédé selon la revendication 2 dans lequel l'étape "a" comprend le transport desdites cellules à travers ledit faisceau laser.

4. Procédé selon la revendication 2 dans lequel l'étape "a" comprend le balayage d'une surface prédéterminée avec ledit faisceau laser à onde continue, et l'étape "b" comprend la mise en place d'un milieu contenant lesdites cellules vivantes et fractions de ladite substance étrangère dans ladite surface prédéterminée.

5. Procédé selon la revendication 2 dans lequel ledit milieu est un liquide approprié pour la culture desdites cellules vivantes.

6. Appareil pour réaliser le procédé de l'une des revendications 1-5 comprenant:

une source laser;

un système optique pour envoyer un faisceau laser à chacune des cellules vivantes sélectionnées;

un écran de contrôle pour présenter l'image de ladite cellule sélectionnée;

un moyen pour déterminer la position dans laquelle se trouve chaque cellule vivante sélectionnée dans le champ dudit écran de contrôle;

un moyen réagissant à un signal provenant dudit moyen de détermination de la position de la cellule pour projeter ledit faisceau laser à partir de ladite source laser et

un moyen réagissant à un signal provenant de ladite position de la cellule;

un moyen de détermination pour diriger ledit faisceau laser vers une cellule vivante restant dans la position représentée par ledit signal.

7. Appareil pour réaliser le procédé de l'une des revendications 1-5 comprenant:

une source laser;

un système optique pour projeter un faisceau laser sur chaque cellule vivante sélectionnée;

un écran de contrôle pour présenter l'image de ladite cellule sélectionnée;

un moyen pour déterminer la position dans laquelle chaque cellule vivante sélectionnée reste dans le champ dudit écran de contrôle;

un moyen de mémoire réagissant aux signaux provenant dudit moyen de détermination de la position de la cellule pour projeter les positions des cellules vivantes sélectionnées et

un moyen pour lire les éléments d'information provenant dudit moyen de mémoire pour diriger ledit faisceau laser vers lesdites cellules sélectionnées l'une après l'autre.

# FIG.1

$10\mu$

# FIG.3

$10\mu$

# FIG.2

(A)

(B)

(C)

# FIG.4

# FIG.5

# FIG.6

# FIG.7

# FIG.8

# FIG.9

# FIG.10

10μ

9